Europäisches Patentamt

European Patent Office

Office européen des brevets

(19)

(11) Numéro de publication : **0 075 336**
**B1**

(12) **FASCICULE DE BREVET EUROPÉEN**

(45) Date de publication du fascicule du brevet :
19.12.84

(21) Numéro de dépôt : 82200972.6

(22) Date de dépôt : 29.07.82

(51) Int. Cl.³ : **C 07 B 27/00,** C 07 D307/36//
C07C43/23, C07C39/19,
C07C15/44, C07C43/215,
C07C11/02, C07C69/618,
C07D307/54

(54) Procédé de transformation d'un aldéhyde en un alcène, en particulier d'aldéhydes phénoliques en alcènes correspondants.

(30) Priorité : 18.09.81 FR 8117825

(43) Date de publication de la demande :
30.03.83 Bulletin 83/13

(45) Mention de la délivrance du brevet :
19.12.84 Bulletin 84/51

(84) Etats contractants désignés :
AT BE CH DE GB IT LI LU NL SE

(56) Documents cités :
FR-A- 2 487 334
TETRAHEDRON LETTERS, no. 30, juillet 1974, pages
2587-2590, Pergamon Press, Oxford (GB);
CHEMICAL ABSTRACTS, vol. 92, no. 1, 7 janvier 1980,
page 569, no. 6038q, Columbus Ohio (USA);
SYNTHESIS, juin 1976, pages 396-398, Stuttgart (DE);
SYNTHESIS, novembre 1979, pages 884-885, Stuttgart (DE);

(73) Titulaire : AGRIFURANE S.A.
Z.I. de Boé Cidex 4412
F-47240 Bon Encontre (FR)

(72) Inventeur : Delmas, Michel
Rouens
F-12140 Entrayges (FR)
Inventeur : Gaset, Antoine
75 Allée de Brienne
F-31000 Toulouse (FR)
Inventeur : Le Bigot, Yves
Le Pons
F-34380 Saint Martin de Londres (FR)

(74) Mandataire : Barre, Philippe
Cabinet Barre, Gatti, Laforgue 95, rue des Amidonniers
F-31069 Toulouse Cedex (FR)

EP 0 075 336 B1

# 0 075 336

## Description

L'invention concerne un procédé de transformation d'un aldéhyde en un alcène correspondant en particulier de type E majoritaire. Elle s'applique en particulier pour la transformation d'aldéhydes phénoliques en alcènes correspondants ou encore de furfural en furfurylidènes diversement substitués. Par aldéhyde, on entend de façon générale tout corps ayant une ou plusieurs fonctions aldéhydes.

La réaction de WITTIG découverte en 1954 est actuellement très utilisée pour préparer des alcènes à partir des aldéhydes correspondants ; en particulier cette réaction permet d'obtenir des alcènes fonctionnalisés ou non en α ou β d'un carbone de la double liaison éthylénique par un cycle ou une chaîne aliphatique, et non fonctionnalisé à partir du même carbone par un hétéroatome ou un groupe fonctionnel hétéroatomique ; on sait que ce type d'alcènes intervient dans de nombreuses synthèses, ou sont eux-mêmes directement actifs dans de nombreux domaines notamment dans l'industrie chimique (polymères, intermédiaires de synthèse...), pharmaceutique (antibactériens, analgésiques...), agrochimique et agroalimentaire (phéromones, parfums, insecticides...). Les alcènes obtenus à partir des aldéhydes phénoliques ou des dérivés du furfural sont particulièrement actifs dans ce dernier domaine, essentiellement par leurs propriétés antibactériennes et antifongiques.

La réaction de WITTIG consiste en une condensation entre un sel de phosphonium et un composé carbonylé en présence de certaines bases fortes et en milieu organique aprotique strictement anhydre ; les bases utilisées sont des bases fortes du type butyl-lithium, hydrures... qui décomposent l'eau (force basique supérieure à celle de l'ion hydroxyde) et les solvants sont des solvants organiques basiques ou peu basiques. L'obtention d'alcènes par cette réaction pose des problèmes techniques très difficiles à résoudre à échelle industrielle. En premier lieu l'état strictement anhydre du milieu réactionnel est difficile à atteindre dans les installations industrielles et exige des traitements et contrôles spéciaux de mise en œuvre très onéreux (étuvage, séchage par courant d'azote...). En outre, les bases fortes utilisées ont une réactivité violente et explosive avec l'eau, qui rend leur stockage dangereux ; de plus, ces produits sont chers et grèvent considérablement le coût de la synthèse. Au surplus, la réaction est généralement mise en œuvre dans des solvants lourds (tels que diméthylsulfoxyde DMSO, diméthyl formamide DMF, hexaméthyl phosphorotriamide HMPT...), qui sont difficiles à séparer des produits réactionnels et à recycler. Enfin, cette réaction qui est opérante avec un rendement satisfaisant avec les aldéhydes aromatiques, devient peu intéressante avec les aldéhydes phénoliques en raison de son faible rendement.

En vue d'éliminer ou de réduire ces défauts, la réaction de WITTIG a fait l'objet de nombreuses études et deux types essentiels de techniques de mise en œuvre ont été développés. L'une des techniques appliquées vers 1975 à la réaction de WITTIG est celle de la catalyse par fransfert de phase (on se reportera par exemple à la publication suivante qui décrit ce type de technique : TETRAHEDRON LETTERS n° 30, 1974 PERGAMON PRESS GB auteurs : W TAGAKI et al) ; cette technique permet d'éviter les difficultés liées à l'état anhydre imposé au milieu, mais implique la présence d'une phase aqueuse importante et corrosive qui limite son intérêt ; de plus, le rendement en alcène est médiocre et la réaction n'est pas stéréosélective (rapport Z/E de l'ordre de 1). Enfin, cette technique n'est pas applicable aux aldéhydes phénoliques et aliphatiques en raison de réactions secondaires qui apparaissent ; cette limitation est un inconvénient grave en pratique car les alcènes phénoliques et aliphatiques sont des substrats extrêmement utiles, qui sont indispensables pour la synthèse de produits actifs, essentiels dans le domaine de la chimie biologique, tels par exemple que les phéromones, les prostaglandines et les antibactériens.

Un autre type de technique utilisant une réaction dite de WITTIG-HORNER, dérivée de la réaction de WITTIG, consiste à faire réagir un phophonate en présence d'une base forte en milieu liquide biphasique, constitué par une phase aqueuse et une phase organique non miscible. (On peut se reporter pour plus de précisions à la publication suivante : revue SYNTHESIS 1976, 396 auteurs : M. MIKOLAJCZYK et al). Une variante décrite en 1979 (revue SYNTHESIS 1979, p 884, auteurs : FOUCAUDX et al) consiste à réaliser la réaction en phases solide/liquide.

Toutefois, comme la technique précédente, ces techniques ne sont pas applicables aux aldéhydes phénoliques et aliphatiques, ce qui représente une grave limitation comme déjà mentionné. De plus, elles ne permettent d'obtenir que des alcènes fonctionnalisés en α ou β du carbone de la double liaison éthylénique provenant des phosphonates, par un hétéroatome ou un groupe fonctionnel hétéroatomique. Ces alcènes sont très différents sur le plan de leur activité des alcènes non fonctionnalisés en α ou β et cette limitation réduit encore l'intérêt de ces procédés.

Par ailleurs, on a décrit dans la demande de brevet n° 80.16767 déposée le 28/07/1980 par la demanderesse un nouveau procédé de transformation d'aldéhydes, qui écarte les inconvénients sus-évoqués des procédés classiques. Ce procédé est essentiellement caractérisé en ce que la réaction de transformation s'effectue en milieu organique aprotique avec un faible taux d'hydratation contrôlé. Ce procédé conduit à une stéréosélectivité importante en faveur des isomères type Z.

La présente invention se propose d'indiquer un procédé relevant d'une idée-mère inventive voisine de celle du procédé ci-dessus et permettant d'éliminer dans les mêmes conditions, les défauts des procédés classiques et de fournir des alcènes de type E majoritaires. On sait que ce type d'isomère est,

2

contrairement à l'isomère Z, actif sur le plan biologique de sorte que son obtention majoritaire est capitale dans de nombreuses applications.

L'invention vise en particulier un procédé permettant d'obtenir des alcènes de ce type fonctionnalisés ou non en α ou β du carbone de la double liaison éthylénique, provenant d'un réactif phosphorique, par un cycle ou une chaîne aliphatique ou un hétéroatome ou un groupe fonctionnel hétéro-atomique.

Un autre objectif de l'invention est d'indiquer un procédé, susceptible d'être mis en œuvre avec des rendements satisfaisants aussi bien avec des aldéhydes phénoliques aromatiques ou hétéroaromatiques qu'avec des aldéhydes aliphatiques, pour obtenir le type d'alcènes sus-évoqués.

Un autre objectif est de supprimer l'exigence stricte du caractère anhydre de la réaction de WITTIG en fournissant un procédé apte à être mis en œuvre en présence d'eau dans des conditions très performantes.

Un autre objectif est de permettre l'utilisation de bases classiques ne présentant aucun risque et bénéficiant de coûts réduits.

Un autre objectif de l'invention est d'indiquer un procédé apte à opérer avec un solvant léger, peu onéreux, susceptible d'être régénéré et duquel les produits de synthèse sont faciles à séparer.

Un autre objectif est d'indiquer un procédé mettant en œuvre une réaction non exothermique, ne présentant aucun danger, même avec de grandes quantités de produits en présence, et se déroulant dans des conditions douces de température et de pression.

Un autre objectif est d'indiquer un procédé permettant d'ajuster de façon précise la stéréosélectivité de la réaction en vue d'obtenir simultanément un alcène du type E majoritaire et un alcène du type Z dans des proportions désirées.

A cet effet, le procédé de transformation visé par l'invention est du type dans lequel l'aldéhyde est mis en présence d'un réactif phosphorique et d'une base, dans un solvant organique ; selon la présente invention, en vue d'obtenir un alcène du type E majoritaire, on réalise la réaction dans les conditions suivantes :

On utilise comme solvant un alcool,

On utilise une base minérale ayant en milieu aqueux une force basique inférieure ou égale à celle de l'ion hydroxyde ou une base organique fortement basique de la famille des amines,

et on limite le taux d'hydratation du milieu réactionnel à une valeur au plus égale à 10 moles d'eau par mole d'aldéhyde.

Les expérimentations ont montré, de façon inattendue, que l'utilisation d'un alcool comme solvant (condition essentielle) conjuguée à la maîtrise du taux d'hydratation permet, en utilisant une base du groupe indiqué, une transformation sélective de l'aldéhyde en alcène de type E majoritaire avec de très hauts rendements. L'utilisation du solvant dans les conditions précitées ne soulève aucune difficulté pratique compte tenu de la banalité de ce corps sans aucun danger et dont le coût est très faible. Le taux d'hydratation est facile à contrôler dans les limites fournies et les conditions difficiles de la réaction de WITTIG sont ainsi éliminées.

Les alcools qui paraissent fournir les meilleurs rendements sont le méthanol et l'éthanol qui seront donc utilisés préférentiellement ; il est à noter que ces alcools sont très faciles à séparer ensuite du milieu réactionnel et à recycler.

Selon un mode de mise en œuvre préféré, la quantité d'alcool est avantageusement ajustée de façon à réaliser une dilution de l'aldéhyde comprise entre environ 0,2 et 2 moles d'aldéhydes par litre d'alcool.

De plus, la base utilisée peut être une base classique notamment un composé ionique de métaux alcalin où l'anion présente un caractère basique en particulier composé ionique de potassium, de rubidium ou de césium ; ce type de base n'entraîne aucun risque sur le plan industriel et son prix de revient est faible.

En outre, on a pu constater que cette réaction est également opérante avec de bons rendements avec les aldéhydes phénoliques, aromatiques, hétéroaromatiques, aliphatiques, fonctionnalisés ou non. Les conditions du procédé permettent l'inhibition des réactions secondaires d'aldolisation, ce qui explique que les rendements obtenus avec les aldéhydes aliphatiques présentent également des valeurs élevées.

Il est à noter que, dans le cas des aldéhydes aromatiques, il se produit parfois dans les procédés classiques une réaction secondaire (dite de CANNIZARO) qui limite le rendement en alcène. Dans le cas du procédé de l'invention, cette réaction est extrêmement peu sensible ; elle est totalement inhibée lorsqu'on utilise comme base le carbonate de potassium, de rubidium ou de césium.

La réaction se produisant dans le procédé de l'invention est légèrement endothermique et ne suscite aucun risque de développement brutal. Le rendement est amélioré par un léger chauffage : en pratique, le milieu réactionnel peut être gardé à température ambiante, la réaction restant opérante d'environ − 30° à + 120 °C.

De plus, sur le plan des quantités relatives de produits, on opère avantageusement dans les conditions suivantes :

base utilisée en léger excès stœchiométrique par rapport à l'aldéhyde.

réactif phosphorique utilisé en léger excès stœchiométrique par rapport à l'aldéhyde.

Le réactif phosphorique utilisé peut être un sel de phosphore phosphonium ou phosphonate et permet d'obtenir un alcène fonctionnalisé ou non en α ou β de la double liaison éthylénique à partir du carbone de celle-ci provenant du sel de phosphore. Ces alcènes ont un intérêt pratique, large et varié en agrochimie, dans les industries pharmaceutiques et chimiques, tant par leurs activités biologiques propres que comme intermédiaires de synthèse ; c'est un avantage important de l'invention que de permettre d'orienter la réaction vers la production de ce type de composé.

Par ailleurs, le procédé conforme à l'invention permet, le cas échéant, d'ajuster la stéréochimie de la réaction, les alcènes de type E obtenus étant toujours majoritaires. Dans ce but, il consiste à additionner au solvant principal constitué par l'alcool un solvant organique aprotique, en particulier dioxanne ou diméthoxyéthane (DME) ou chlorure de méthylène ou nitrobenzène. La proportion des deux solvants fixe la stéréochimie de la réaction qui peut ainsi être adaptée à l'application envisagée.

L'invention exposée de façon générale ci-dessus est illustrée ci-après par des exemples non limitatifs de mise en œuvre.

## Exemple 1

Synthèse du hydroxy-1, méthoxy-2, (pentène-1) yl-4 benzène à partir de la vanilline en présence de carbonate de potassium et de bromure de butyl-triphényl-phosphonium dans le méthanol.

On place dans un réacteur de 1 litre : 0,12 mole de sel de phosphonium, 0,12 mole de carbonate de potassium, 0,1 mole d'aldéhyde dans 100 ml de méthanol commercial.

Après 1 heure de réaction à température ambiante, le milieu réactionnel et filtré et concentré. L'alcène formé est obtenu après distillation (p. eb : 90°/0,5 mm Hg) avec un rendement voisin de 85 % et caractérisé par ses spectres infra-rouge et R.M.N.

La réaction est stéréosélective et l'isomère E est obtenu majoritairement ( % isomère E/ % isomère Z : 85/15). Aucun autre procédé connu ne permet d'obtenir un tel résultat.

Les aldéhydes phénoliques qui peuvent être ainsi valorisés sont pour la plupart extraits de la matière végétale notamment par traitement biologique ou chimique de la lignine.

## Exemple 2

Synthèse du hydroxy-1, méthoxy-2, (heptène-1) yl-4 benzène à partir de la vanilline en présence de fluorure de potassium et de bromure d'hexyl-triphénylphosphonium dans le méthanol.

Dans un réacteur identique de 1 litre, on place 0,12 mole de sel de phosphonium, 0,12 mole de fluorure de potassium, 0,1 mole d'aldéhyde dans 100 ml de méthanol commercial.

Après 2 heures 15 d'agitation à température ambiante ; l'hydroxy-1, méthoxy-2, (heptène-1) yl-4 benzène est obtenu pur, avec un rendement de 88 %, par distillation sous vide (p.eb. 115 °C/0,8 mm Hg).

La stéréosélectivité de la réaction conduit au produit majoritairement E (% isomère E/ % isomère Z : 87/13).

L'activité antibactérienne de ce produit est mise en évidence par son coefficient phénolique à 37 °C :

625 sur St Aurens
667 sur M. Tuberculosis
556 sur C. Albicans.

## Exemple 3

Synthèse de l'isoengénol à partir de la vanilline en présence de carbonate de potassium et de bromure d'éthyle triphényl phosphonium dans le méthanol commercial.

Dans un réacteur de 250 ml, on place 0,025 mole de sel de phosphonium, 0,025 mole de carbonate de potassium, 0,02 mole d'aldéhyde et 20 ml de méthanol commercial.

Le milieu est mis en agitation soit pendant

5 heures à − 10 °C
2 heures 30 à 0 °C
1 heure à 20 °C
0 heure 30 à 40 °C.

Ces conditions permettent la transformation complète de la vanilline en isoeugénol. Après distillation, l'isoeugénol est identifié par comparaison de ses caractéristiques physicochimiques avec celles du produit commercial.

## Exemple 4

Synthèse du hydroxy-1, (butène-1)yl-2 benzène à partir de l'aldéhyde salicylique en présence de carbonate de césium et de bromure de propyl-triphényl phosphonium dans le méthanol.

On mélange dans un réacteur, 0,025 mole de sel de phosphonium, 0,025 mole de carbonate de césium et 0,02 mole d'aldéhyde dans 20 ml de méthanol commercial.

Le mélange réactionnel est agité à température ambiante pendant 1 heure 30. L'hydroxy-1, (butène-1)

**0 075 336**

yl-2 benzène ( % isomère E/ % isomère Z : 82/18) est obtenu pur avec un rendement de l'ordre de 80 % par distillation (p. eb. : 75 °C/l mm Hg). Il est caractérisé par ses spectres infra-rouge et de résonance magnétique nucléaire dont les résultats sont confirmés par micro-analyse.

Exemple 5

Synthèse de hydroxy-1, vinyl-4 benzène à partir de l'hydroxy-4 benzaldéhyde en présence de carbonate de potassium et de bromure de méthyl-triphénylphosphonium dans le méthanol.

Dans un réacteur de 250 ml, on place : 0,025 mole de sel de phosphonium, 0,025 mole de carbonate de potassium et 0,02 mole d'hydroxy-4 benzaldéhyde dans 20 ml de méthanol commercial.

Le milieu réactionnel est agité à température ambiante pendant 4 heures. La majeure partie de l'oxyde de triphénylphosphine formé précite et est séparé par filtration de la phase organique qui est ensuite analysée par chromatographie en phase vapeur sur colonne du type OV 101. L'hydroxy-1 vinyl-4 benzène est obtenu avec un rendement voisin de 90 % après distillation.

Exemple 6

Synthèse du (butène-1)yl-2 furanne à partir de furanne 2 carboxaldéhyde en présence de carbonate de potassium et de bromure de propyl-triphényl phosphonium dans l'éthanol.

On place dans un réacteur de 250 ml : 0,025 mole de sel de phosphonium, 0,025 mole de carbonate de potassium et 0,02 mole d'aldéhyde dans 20 ml d'éthanol à 95°.

Le mélange réactionnel est agité au reflux de l'éthanol pendant 2 heures. Ces conditions permettent la transformation complète du furfural en (butène-1)yl-2 furanne qui est extrait après filtration et concentration du milieu réactionnel par chromatographie sur une courte colonne de silicagel en utilisant l'hexane comme éluant.

La réaction est stéréosélective et l'isomère E est obtenu majoritairement (% isomère E/% isomère Z : 70/30).

Le composé de départ : le furanne 2-carboxaldéhyde (ou furfural) est fabriqué industriellement à partir de sous-produits agricoles riches en pentosanes (rafles de maïs, balles de riz et d'avoine, bagasses de canne à sucre, coques d'arachides et déchets de bois). Ces déchets traités en milieu acide concentré conduisent à des aldopentoses obtenus par hydrolyse de leurs pentosanes qui se déshydratent pour donner le furfural.

Exemple 7

Synthèse du (pentène-1)yl-2 furanne à partir du furanne-2 carboxaldéhyde en présence de carbonate de potassium et de bromure de butyl-triphénylphosphonium dans le méthanol.

Dans un réacteur identique de 250 ml on place 0,025 mole de sel de phosphonium, 0,025 mole de carbonate de potassium et 0,02 mole de furfural dans 20 ml de méthanol commercial.

Le mélange réactionnel est porté au reflux et maintenu sous agitation pendant 1 heure 15. Le (pentène-1)yl-2 furanne (% isomère E/% isomère Z : 69/31) est obtenu pur avec un rendement de l'ordre de 85 % après filtration et chromatographie rapide sur gel de silice.

Exemple 8

Synthèse du (pentène-1)yl benzène à partir de benzaldéhyde en présence de carbonate de potassium et de bromure de butyl-triphényl phosphonium dans un mélange de dioxanne-1,4 et de méthanol.

On place dans un réacteur : 0,025 mole de sel de phosphonium, 0,025 mole de carbonate de potassium et 0,02 mole de benzaldéhyde dans 20 ml de mélange de dioxanne-1,4 et de méthanol du commerce.

Après 2 heures 30 d'ébullition, sous agitation, le milieu réactionnel est filtré pour éliminer la majeure partie d'oxyde de triphénylphosphine formé. Après évaporation du solvant, le (pentène-1) yl benzène est chromatographié sur une courte colonne de gel de silice ; éluant ; hexane, quelque soit le pourcentage de méthanol dans le mélange de solvant, le rendement en oléfine est voisin de 95 %.

La stéréosélectivité de la réaction est fonction de la quantité de méthanol dans le milieu.

pour 1 ml de méthanol, 30 % d'isomère E
pour 3,5 ml de méthanol, 50 % d'isomère E
pour 5 ml de méthanol, 64 % d'isomère E
pour 20 ml de méthanol, 77 % d'isomère E.

Exemple 9

Synthèse de l'anéthole à partir de l'anisaldéhyde en présence de carbonate de rubidium et de bromure d'éthyl-triphényl phosphonium dans le méthanol.

5

# 0 075 336

A 0,025 mole de sel de phosphonium et 0,025 mole de carbonate de rubidium, on ajoute 0,02 mole d'anisaldéhyde et 20 ml de méthanol du commerce.

Le mélange réactionnel est agité au reflux du méthanol pendant 3 heures. L'anéthole (% isomère E/% isomère Z : 67/33) est obtenu pur avec un rendement de l'ordre de 85 % et identifié par comparaison avec le produit commercial et analyse de ses spectres infra-rouge et R.M.N.

Le trans-anéthole, produit majoritaire de cette synthèse, présente un caractère insecticide notoire et est un corps très important dans le domaine de l'agro-alimentaire.

## Exemple 10

Synthèse du tétradécène-5 à partir de décanal en présence de carbonate de potassium et de bromure de butyl-triphénylphosphonium dans le butanol-1.

On mélange dans un réacteur : 0,025 mole de sel de phosphonium, 0,025 mole de carbonate de potassium, 0,02 mole d'aldéhyde et 0,04 mole d'eau dans 20 ml de butanol-1.

Le milieu réactionnel est porté au reflux et maintenu sous agitation pendant 3 heures 30. L'alcène correspondant est obtenu après chromatographie sur colonne avec un rendement de 70 % et caractérisé par ses spectres infra-rouge et de résonnance magnétique nuclaire et par micro-analyse.

La réaction est stéréosélective et l'isomère E est obtenu majoritairement (% isomère E/% isomère Z : 70/30).

La séparation de ces isomères peut être obtenue par chromatographie sur colonne d'alumine imprégnée de 20 % de nitrate d'argent.

Il faut souligner que dans ces conditions expérimentales, la réaction secondaire d'aldolisation est inhibée.

## Exemple 11

Synthèse du phényl-3 propénoate d'éthyle à partir de benzaldéhyde en présence de carbonate de potassium et de diméthylphosphonoacétate d'éthyle dans l'éthanol.

On mélange dans un réacteur 0,025 mole de phosphonate, 0,025 mole de carbonate de potassium et 0,02 mole d'aldéhyde dans 20 ml d'éthanol commercial.

Le mélange réactionnel est agité au reflux de l'éthanol pendant 1 heure. Ces conditions permettent la transformation complète du benzaldéhyde en phényl-3 propénoate d'éthyle qui est extrait après filtration et concentration du milieu réactionnel par chromatographie sur colonne de gel de silice en utilisant un mélange d'hexane et d'éther comme éluant.

La réaction est stéréosélective et l'isomère E est obtenu majoritairement (% isomère E/% isomère Z : 86/14).

Le rendement de la réaction après purification est de l'ordre de 85 %. Le phényl-3 propénoate d'éthyle est caractérisé par ses spectres infra-rouge et de résonance magnétique nuclaire dont les résultats sont confirmés par microanalyse.

## Exemple 12

Synthèse du furyl-3 propénoate d'éthyle à partir de furfural en présence de carbonate de potassium et de diméthylphosphonoacétate d'éthyle dans l'éthanol.

On place dans un réacteur de 250 ml : 0,025 mole de sel de phosphonium, 0,025 mole de carbonate de potassium, 0,02 mole d'aldéhyde dans 20 ml d'éthanol à 95.

Le mélange réactionnel est porté au reflux et maintenu sous agitation pendant 1 heure. Le furyl-3 propénoate d'éthyle (% isomère E/% isomère Z : 90/10) est obtenu pur avec un rendement de l'ordre de 80 % après filtration et chromatographie sur gel de silice.

Il est à noter que la stéréochimie de l'alcène obtenu à partir des phosphonates est indépendante de la nature du solvant, que la réaction soit effectuée dans le dioxanne, dans l'éthanol ou dans un mélange de ces deux solvants, la stéréochimie préférentielle E ne change pas.

## Revendications

1. Procédé de transformation d'un aldéhyde en alcène correspondant dans lequel l'aldéhyde est mis en présence d'un réactif phosphorique et d'une base dans un solvant organique, ledit procédé étant caractérisé en ce que, en vue d'obtenir un alcène du type E majoritaire :

on utilise comme solvant un alcool,

on utilise une base minérale ayant en milieu aqueux une force basique inférieure ou égale à celle de l'ion hydroxyde ou une base organique, fortement basique de la famille des amines,

et on limite le taux d'hydratation du milieu réactionnel à une valeur au plus égale à environ 10 moles d'eau par mole d'aldéhyde.

6

2. Procédé de transformation selon la revendication 1, caractérisé en ce qu'on utilise comme solvant du méthanol ou de l'éthanol.

3. Procédé de transformation selon l'une des revendications 1 ou 2, caractérisé en ce que l'on utilise comme base un composé ionique de métaux alcalins où l'anion présente un caractère basique, en particulier composés ioniques de potassium, rubidium ou césium.

4. Procédé de transformation selon l'une des revendications 1, 2 ou 3, caractérisé en ce qu'il est mis en œuvre à température ambiante.

5. Procédé de transformation selon l'une des revendications 1, 2, 3 ou 4, caractérisé en ce que la quantité d'alcool est ajustée de façon à réaliser une dilution de l'aldéhyde comprise entre environ 0,2 et 2 moles d'aldéhyde par litre d'alcool.

6. Procédé de transformation selon l'une des revendications 1, 2, 3, 4 ou 5, permettant d'obtenir un alcène fonctionnalisé ou non en α ou β de la double liaison éthylénique, caractérisé en ce que l'on utilise comme réactif phosphorique un sel de phosphore (phosphonium ou phosphonate)

7. Procédé de transformation selon l'une des revendications 1, 2, 3, 4, 5 ou 6, dans lequel le réactif phosphorique est mis en présence d'un léger excès stœchiométrique par rapport à l'aldéhyde.

8. Procédé de transformation selon l'une des revendications 1, 2, 3, 4, 5, 6 ou 7, caractérisé en ce que, en vue d'obtenir simultanément un alcène du type E majoritaire et un alcène du type Z avec une stéréochimie désirée, on additionne au solvant principal constitué par l'alcool un solvant organique aprotique, en particulier dioxanne, ou diméthoxyéthane (DME) ou chlorure de méthylène ou nitrobenzène.

9. Procédé selon l'une des revendications précédentes, appliqué à la transformation d'un aldéhyde phénolique, en particulier extrait de la lignine, dans lequel on utilise comme base une base minérale autre que la soude et la potasse.

10. Procédé selon l'une des revendications 1 à 8, appliqué à la transformation du furfural en furfurylidène.

## Claims

1. Process for the conversion of an aldehyde into a corresponding alkene, in which the aldehyde is brought into contact with a phosphorus reagent and an organic base in an organic solvent, said process being characterised in that, with a view to obtaining an alkene predominantly of type E,

an alcohol is used as solvent,

an inorganic base, having a basic strength less than, or equal to, that of the hydroxyl ion in aqueous medium, or a strongly basic organic base from the amine group is used ; and

the water content of the reaction medium is limited to a value that is, at most, equal to about 10 Mols of water per Mol of aldehyde.

2. Conversion process according to Claim 1, characterised in that the solvent used is methanol or ethanol.

3. Conversion process according to either Claim 1 or 2, characterised in that the base used is an ionic alkali metal compound in which the anion has a basic character, especially ionic compounds of potassium, rubidium or caesium.

4. Conversion process according to one of the Claims 1, 2 or 3, characterised in that it is carried out at ambient temperature.

5. Conversion process according to one of the Claims 1, 2, 3 or 4, characterised in that the quantity of alcohol is adjusted so as to bring about a dilution of the aldehyde of between about 0.2 and 2 Mols of aldehyde per litre of alcohol.

6. Conversion process according to one of the Claims 1, 2, 3, 4 or 5, enabling an alkene, substituted or not in the α-position or β-position of the ethylenic double bond, to be obtained, characterised in that the phosphorus reagent used is a phosphorus salt (phosphonium or phosphonate).

7. Conversion process according to one of the Claims 1, 2, 3, 4, 5 or 6, in which the phosphorus reagent is employed in slight stoichiometric excess, relative to the aldehyde.

8. Conversion process according to one of the Claims 1, 2, 3, 4, 5, 6 or 7, characterised in that, with a view to obtaining simultaneously a type E alkene predominantly and a type Z alkene having a desired stereochemistry, an aprotic organic solvent, especially dioxan or dimethoxyethane (DME) or methylene chloride or nitrobenzene, is added to the main solvent, consisting of the alcohol.

9. Process according to one of the preceding claims, applied to the conversion of a phenolic aldehyde, especially lignin extract, in which the base used is an inorganic base other than caustic soda or caustic potash.

10. Process according to one of the Claims 1 to 8, applied to the conversion of furfural into a furfurylidene.

## Ansprüche

1. Verfahren zur Umsetzung von einem Aldehyd in ein übereinstimmendes Alken, bei welchem das Aldehyd mit einem Phosphor-Reagens und einer Base in einem organischen Lösungsmittel zusammengebracht wird, wobei besagtes Verfahren dadurch gekennzeichnet ist, dass man, um vorwiegend ein Alken des E-Typs zu erhalten,

als Lösungsmittel einen Alkohol verwendet,

eine anorganische Base mit einer basischen Stärke in wässrigem Medium, welche geringer als die des Hydroxylions oder ihr gleich ist, oder eine stark basische organische Base aus der Gruppe der Amine verwendet und

den Wassergehalt des Reaktionsmediums auf einen Wert begrenzt, welcher höchstens etwa 10 Molen Wasser pro Mol Aldehyd gleich ist.

2. Umsetzungsverfahren nach Anspruch 1, dadurch gekennzeichnet, dass man als Lösungsmittel Methanol oder Äthanol verwendet.

3. Umsetzungsverfahren nach einem der Ansprüche 1 oder 2, dadurch gekennzeichnet, dass man als Base eine ionische Alkaliverbindung verwendet, in welcher das Anion basischen Charakter aufweist, insbesondere ionische Kalium-, Rubidium- oder Caesiumverbindungen.

4. Umsetzungsverfahren nach einem der Ansprüche 1, 2 oder 3, dadurch gekennzeichnet, dass es bei Umgebungstemperatur durchgeführt wird.

5. Umsetzungsverfahren nach einem der Ansprüche 1, 2, 3 oder 4, dadurch gekennzeichnet, dass die Alkoholmenge so eingestellt wird, dass eine Verdünnung des Aldehyds zwischen 0,2 und 2 Molen Aldehyd pro Liter Alkohol hergestellt wird.

6. Umsetzungsverfahren nach einem der Ansprüche 1, 2, 3, 4 oder 5, wobei es ermöglicht wird, ein Alken zu erhalten, welches in der $\alpha$- oder $\beta$-Stellung der äthylenischen Doppelbindung substituiert oder nicht substituiert ist, dadurch gekennzeichnet, dass man als Phosphor-Reagens ein Phosphorsalz (Phosphonium- oder Phosphonat) verwendet.

7. Umsetzungsverfahren nach einem der Ansprüche 1, 2, 3, 4, 5 oder 6, bei welchem das Phosphor-Reagens in geringem stöchiometrischem Überschuss im Verhältnis zum Aldehyd eingesetzt wird.

8. Umsetzungsverfahren nach einem der Ansprüche 1, 2, 3, 4, 5, 6 oder 7, dadurch gekennzeichnet, dass man, um ein Alken des E-Typs vorwiegend und ein Alken des Z-Typs mit einer gewünschten Stereochemie gleichzeitig zu erhalten, dem aus dem Alkohol bestehenden Haupt-Lösungsmittel ein aprotides organisches Lösungsmittel, insbesondere Dioxan oder Dimethoxyäthan (DMÄ) oder Methylenchlorid oder Nitrobenzol, zusetzt.

9. Verfahren nach einem der vorangehenden Ansprüche unter Anwendung auf die Umsetzung eines phenolischen Aldehyds, insbesondere Lignin-Extrakt, bei welchem man als Base eine andere anorganische Base als Natron- und Kalilauge verwendet.

10. Verfahren nach einem der Ansprüche 1 bis 8, unter Anwendung auf die Umsetzung des Furfurals in ein Furfuryliden.